# EUROPÄISCHE PATENTSCHRIFT

(11) **EP 4 753 597 B1**
(45) Veröffentlichungstag und Bekanntmachung des Hinweises auf die Patenterteilung: **30.09.2026**
(21) Anmeldenummer: 25787343.0
(22) Anmeldetag: 29.09.2025
(51) Int. Cl.: A61B 17/80

(54) **SYSTEM MIT ANTERIORER ZERVIKALER PLATTE UND SCHRAUBEN-VERRIEGELUNGSELEMENT**
SYSTEM WITH ANTERIOR CERVICAL PLATE AND SCREW LOCKING ELEMENT
SYSTÈME AVEC PLAQUE CERVICALE ANTÉRIEURE ET ÉLÉMENT DE VERROUILLAGE DE VIS

(30) Priorität: 01.10.2024 DE 102024128445
(43) Veröffentlichungstag der Anmeldung: 10.06.2026
(73) Patentinhaber: AESCULAP AG, 78532 Tuttlingen (DE)
(72) Erfinder: STERN, Andreas, 78087 Mönchweiler (DE); SCHULTZ, Robert, 78532 Tuttlingen (DE); LINDNER, Stephan, 78573 Wurmlingen (DE)
(74) Vertreter: Winter, Brandl - Partnerschaft mbB
(86) Internationale Anmeldenummer: PCT/EP2025/077847
(87) Internationale Veröffentlichungsnummer: WO 2026/073869

(56) Entgegenhaltungen:
- US-A1- 2005 075 633
- US-A1- 2007 043 369
- US-A1- 2009 062 863
- US-A1- 2012 158 068
- US-A1- 2018 199 965
- US-A1- 2023 310 041

## Beschreibung

### Technisches Gebiet

Die vorliegende Offenbarung betrifft ein System mit anteriorer zervikaler Platte mit zumindest einem Verriegelungselement für eine darin eingesetzte Knochenschraube. Solche Systeme dienen zur mono- und multisegmentalen Stabilisierung der Halswirbelsäule.

Es sind verschiedene zervikale Plattensysteme auf dem Markt, die sich unter anderem hinsichtlich ihrer Verriegelungsmechanismen für die eingesetzten Knochenschrauben unterscheiden. Solche Plattensysteme werden bereits von der Anmelderin unter dem Namen Quintex und beispielsweise von den Firmen Bricon (unter dem Namen Shark CP Pro), Zimmer (Trinica), Medtronic (Zevo), Stryker (0zark), Biomet (Maxan), DePuy Synthes (Skyline), GlobusMedical (Assure), Nuvasive (C360) und Biomed (Quantum) vertrieben.

Bei dem zervikalen Plattensystem gemäß US 8,906,077 B2 bzw. EP 2 217 163 B1 kommt ein Sperrelement in Verbindung mit einem gesonderten Fixierelement zum Einsatz.

Bei dem System gemäß US 7909,859 B2 werden die Schrauben mittels Klammern vor dem Herauswandern gesichert.

Bei dem System nach US 8,500,737 B2 wird entweder ein in einer Längsführung verschiebbarer Schnappring oder eine verschiebbare Platte für die Sicherung der eingesetzten Knochenschraube verwendet.

Die aus dem Dokument US 10,492,836 B2 bekannte zervikale Platte ist mit einer Verriegelung in der Ausgestaltung einer Art Flügelschraube, die mittels einer von der Rückseite der zervikalen Platte eingesetzten Spannmutter an der zervikalen Platte fixiert ist. Ein ähnliches Verriegelungselement ist beim System gemäß US8,480,717 B2 verwendet.

Ein Schraubensicherungssystem gemäß DE 202 21 560 U1 verwendet eine auf der Unterseite des betreffenden Schraubenkopfs verschiebbare Scheibe mit Langloch.

Das Verriegelungssystem gemäß US 8,778,001 B2 sieht Verriegelungsplatten vor, die mittels federnder Spreizbeine in einer hinterschnittenen Nut der zervikalen Platte gefangen sind.

Das Plattensystem gemäß US 8,747,441 B2 verwendet als Verriegelungselemente federnde C-Ringe oder geschlitzte Ringe, die in einer entsprechenden Nut der zervikalen Platte aufgenommen sind.

Die Verriegelung gemäß US 8,702,766 B2 verwendet pro Schraube einen verdrehbaren Plattenkörper mit einem radial und in Umfangsrichtung auskragenden Federarm, an dessen distalem Ende ein Klauenkörper sitzt. Der Klauenkörper wird zur Verriegelung beim Verdrehen des Plattenkörpers unter Biegeverformung des Federarms in eine hinterschnittene Führungsnut in der Platte gezwängt und schnappt bei Erreichen der Verriegelungsposition in eine Ausnehmung der Platte. Dieses Konzept verlangt aufgrund der Hinterschneidung eine größere Plattendicke.

Das zervikale Plattensystem gemäß US11,166,755 B2 sichert zwei benachbarte Knochenschrauben mittels über den betreffenden Schraubenkopf schnappbarer Federringe, die mit einer seitlich zwischen benachbarten Schraubenlöchern gelagerten Stellschraube aufweitbar sind.

Bei dem aus dem Dokument EP 1 737 365 B1 bekannten zervikalen Plattensystem ist das Verriegelungselement für die Schrauben von einer federnden Platte gebildet, die in einer Längsführung aufgenommen ist.
Auch in den Dokumenten US 2012/158068 A1, US 2005/075633 A1, US 2009/062863 A1, US 2023/310041 A1 und US 2018/199965 A1 sind Systeme mit anteriorer zervikaler Platte mit Aufnahmedurchbrüchen für Knochenschrauben und einer Verriegelung für eingesetzte, mit einem Wirbelkörper verschraubte und die zervikale Platte gegen den Wirbelkörper drückende Knochenschrauben bekannt, wobei die Verriegelung einen plattenartigen, an der zervikalen Platte beweglich gehaltenen Verriegelungskörper aufweist, der aus einer einen Aufnahmedurchbruch möglichst frei zugänglich lassenden Position in eine Verriegelungsposition bringbar ist, in der der im Aufnahmedurchbruch liegende Schraubenkopf vom Verriegelungskörper abgedeckt ist und so die Migration der Schraube aus der zervikalen Platte heraus verhindert wird.

Bei dem System gemäß US 2007/043369 A1 ist der Verriegelungskörper auf der Unterseite der zervikalen Platte montiert und er verriegelt den Schraubenkopf durch einen Eingriff zwischen Schraubenkopf und Gewinde.
Aus dem Dokument US 2005/075633 A1 ist ein gattungsbildendes zervikales Plattensystem gemäß dem Oberbegriff des Patentanspruchs 1 bekannt. Der Verriegelungskörper ist von einer drehbar um eine senkrecht auf der zervikalen Platte stehende Drehachse an der anterioren zervikalen Platte festgelegten dünnen Scheibe gebildet, die zumindest einen Verriegelungsarm und einen in der Scheibenebene liegenden Stützabschnitt aufweist, der durch Verdrehen der Scheibe um einen vorbestimmten Drehwinkel in eine durch Gegendrehung der Scheibe wieder aufhebbare Rastposition schnappt. Allerdings ist der Stützabschnitt zum Verriegelungsarm nicht über den Umfang winkelmäßig verteilt, und die Scheibe muss zum Erreichen der Rastposition einer Biegebeanspruchung unterworfen werden.

Der Anmeldung liegt die Aufgabe zugrunde, ein gattungsbildendes System mit anteriorer zervikaler Platte und Verriegelung für eingesetzte Knochenschrauben zu schaffen, das einfach zu bedienen ist und größere Flexibilität bei der Gestaltung der zervikalen Platte und eine verbesserte Steuerung des Verriegelungsmoments bietet.

Diese Aufgabe wird mit dem System gemäß Patentanspruch 1 gelöst.

Anmeldungsgemäß ist der Verriegelungskörper nach wie vor von einer drehbar um eine im Wesentlichen senkrecht auf der zervikalen Platte stehende Drehachse an der anterioren zervikalen Platte festgelegten dünnen Scheibe gebildet, die allerdings über den Umfang winkelmäßig verteilt zumindest einen Verriegelungsarm und einen in der Scheibenebene liegenden Stützabschnitt aufweist, der durch Verdrehen der Scheibe um einen vorbestimmten Drehwinkel in eine durch Gegendrehung der Scheibe wieder aufhebbare Rastposition schnappt. Der Stützabschnitt wirkt mit einem auf der zervikalen Platte angebrachten Rastpin zusammen, der beim Verdrehen der Scheibe den Stützabschnitt vor Erreichen der Rastposition verformt, was zu einer besonders platzsparenden Verriegelung führt Der Widerstand des Rastmechanismus lässt sich besonders gut steuern, weil der Stützabschnitt von einem im Wesentlichen kreisbogenförmig verlaufenden Scheibenringausschnitt bzw. -segment gebildet ist, der beim Verdrehen der Scheibe vom Rastpin in radialer Richtung verformbar ist und eine Rastmulde für den Rastpin ausbildet. Die Verformbarkeit kann dabei rein biegeelastisch oder elastisch-plastisch ausgestaltet sein. Die Auslenkung bzw. Deformation des Rastmechanismus funktioniert somit in radialer Richtung. Die federnde Komponente lässt sich auf diese Weise bei geringem Platzbedarf verhältnismäßig lang ausbilden, was insbesondere Toleranzen an der Platte sowie dem Verriegelungsarm ausgleichen kann, ohne dass das Verriegelungsmoment dadurch allzu negativ beeinflusst wird.

Das Öffnen der Verriegelungsarme ist durch eine dem Schließen entgegengesetzte Drehung einfach, um eine möglichst einfache Revision zu ermöglichen. Die Besonderheit des anmeldungsgemäßen Systems liegt also darin, dass eine anteriore zervikale Platte mit drehbaren Verriegelungselementen mit einem integrierten, gegebenenfalls elastischen Rastmechanismus für die Knochenschrauben ausgestattet ist. Dadurch ist die Anordnung so getroffen, dass das Verriegeln bzw. der Antrieb der dünnen Scheibe zu keiner Funktionsbeeinträchtigung des zervikalen Plattensystems führt. Die Dicke der Platte bleibt mit dieser Gestaltung des Verriegelungskörpers unbeeinflusst.

Das System ist damit sowohl für sogenannte Hybride Platten für sogenannte *constrained* Versorgungen als auch für sogenannte Dynamische Platten für dynamische Versorgungen gleichermaßen gut geeignet. Von *constrained* Versorgungen spricht man bei einer eingeschränkten Stabilität/Steifigkeit der Verbindung zwischen zervikaler Platte und Schraube, von dynamischen Versorgungen bei rotatorischer und/oder translatorischer Beweglichkeit zwischen Platte und Schraube. Wegen des Rastmechanismus ist der Verriegelungszustand nicht nur optisch durch die Stellung des zumindest einen Verriegelungsarms, sondern auch haptisch durch den Rasteffekt, gut zu erkennen, was der Bedienungssicherheit weiter zugutekommt. Weil der Rastmechanismus deformierbar ist, kann die Verriegelung nicht nur mit geringer Handkraft geöffnet und geschlossen werden, sondern es ist auch ein mehrfaches Öffnen und Schließen möglich, ohne dass das Verriegelungsmoment wesentlich bzw. spürbar abfällt. Darüber hinaus ergibt sich weitere besondere Vorteil, dass die dünne Platte, welche den Verriegelungskörper ausbildet, die Gesamtstärke des zervikalen Plattensystems bei geringstmöglicher Schwächung der zervikalen Platte nicht wesentlich vergrößert, wodurch es gelingt, Irritationen im Bereich der Halswirbelsäule besser zu vermeiden.

Grundsätzlich kann der Stützabschnitt, mit dem der Rastpin zusammenwirkt, an verschiedensten Stellen der Scheibe vorgesehen sein.

Wenn der Verriegelungskörper im Wesentlichen mittig zwischen zumindest zwei Aufnahmedurchbrüchen für die Knochenschrauben angeordnet und/oder gelagert ist, wird die zervikale Platte von der Lagerung der die Verriegelungsarme tragenden Scheibe geringstmöglich geschwächt, so dass die zervikale Platte mit noch geringerer Dicke ausgeführt werden kann.

Es hat sich gezeigt, dass die den Verriegelungskörper bildende dünne, vorzugsweise eine Dicke im Bruchteilbereich eines Millimeters aufweisende Scheibe bei geeigneter Materialauswahl mit bis zu vier Verriegelungsarmen ausgestattet werden kann, ohne die Funktionsabschnitte des Verriegelungskörpers, d.h. die Verriegelungsarme und den Rastmechanismus mechanisch zu überlasten. Zur sicheren Verriegelung der gesetzten Knochenschrauben kann es genügen, die Knochenschraubenköpfe nur teilweise abzudecken. Es ergibt sich dadurch der zusätzliche Vorteil, dass der Antrieb der Schrauben auch bei geschlossener Verriegelung zugänglich bleiben kann, um ein Nachziehen zu ermöglichen.

Vorteilhafterweise hat der Rastpin von der Drehachse der Scheibe einen Radialabstand, der nur einen Bruchteil der Länge des Verriegelungsarms ausmacht. Mit anderen Worten ist damit der Rastmechanismus möglichst in der Nähe der Drehachse des Verriegelungselementes angeordnet, um ein Aushebeln durch die Schrauben zu vermeiden. Bei einer radialen Erstreckung eines Verriegelungsarms von 3 bis 4 mm hat der Rastpin beispielsweise lediglich einen Radialabstand zwischen 1,5 und 2 mm.

Der Drehwinkel der Scheibe aus der einen Aufnahmedurchbruch frei zugänglich lassenden Position in die Verriegelungsposition kann in weiten Grenzen variiert werden. Versuche haben gezeigt, dass bei Drehwinkeln im Bereich zwischen 10° und 180° die Verriegelungs- und Rastfunktion dauerhaft sichergestellt sind. Bei größeren Drehwinkeln wird der Stützabschnitt in Form eines im Wesentlichen kreisbogenförmig verlaufenden Scheibenringausschnitts bzw. -segments besonders lang, wodurch die Nachgiebigkeit des Rastmechanismus noch besser gesteuert werden kann.

Versuche haben gezeigt, dass die vorstehend beschriebenen Verriegelungs- und Rastfunktionen mehrfach wiederholbar schon dann sichergestellt werden können, wenn die Scheibe mit einer Dicke im Bereich zwischen 0,25 und 0,75 mm ausgeführt ist. Dadurch wird das System insgesamt sehr flach, was zusätzlich zur Vermeidung gegebenenfalls auftretender Gewebeirritationen bzw. von Schluckbeschwerden beiträgt.

Mit dem vorstehend beschriebenen Aufbau gelingt es, die Scheibe mit Handkraft, vorzugsweise mittels eines Bedienwerkzeugs, wie z.B. eines Schraubendrehers in die und aus der Verriegelungsposition zu bringen, was für die Operationstechnik von Vorteil ist, bei der ohnehin Schraubwerkzeuge für die Betätigung der Knochenschrauben verwendet werden. Dabei hat sich gezeigt, dass schon kleine Antriebsprofile in der Scheibe geeignet sind, um das dabei erforderliche Drehmoment sicher zu übertragen, beispielsweise Antriebsprofile mit Abmessungen im Bereich von unter 2 mm, beispielsweise 1,5 mm.

Besondere herstellungs- und bedienungstechnische Vorteile ergeben sich dann, wenn die Scheibe über einen vorzugsweise einstückig angeformten und in einer Bohrung der zervikalen Platte mit Spielpassung aufgenommenen, hohlzylinderförmigen Lagerzapfen, dessen Wandstärke sich zu seinem der Rückseite der zervikalen Platte zugewandten Endabschnitt zunehmend verjüngt und dort mit der zervikalen Platte vorzugsweise über eine Anfasung der Bohrung verstemmt ist, ein Drehlager für die Scheibe ausbildet. Die Verstemmung zwischen zervikaler Platte und Verriegelungskörper ist besonders kostengünstig, wobei die Umformung bzw. plastische Verformung des Verriegelungskörpers beim Verstemmen durch die Fase an der Unterseite der zervikalen Platte gut definiert und begrenzt werden kann. Außerdem sind auf diese Weise die Verriegelungskörper automatisch kanülliert, um einem temporären Fixationspin das Fixieren der zervikalen Platte an einem Wirbelkörper zu ermöglichen.

Gleichzeitig kann der hohlzylinderförmige Abschnitt des Verriegelungskörpers mit einem Antriebsprofil, vorzugsweise mit einer Mehrkantausnehmung für den Eingriff eines Bedienwerkzeugs ausgestattet sein. Diese Mehrkantausnehmung kann sich über eine Länge erstrecken, die im Wesentlichen der Dicke der zervikalen Platte entspricht, so dass ihre Schlüsselweite sehr klein gehalten sein kann, ohne übermäßigen Verschleiß befürchten zu müssen.

Wenn die Mehrkantausnehmung eine Weite erhält, die kleiner ist als die Weite eines Klemmkonus am Schraubwerkzeug für die Knochenschraube bzw. eines Klemmbereichs (Innenkonus) der zugehörigen Knochenschrauben, so ergibt sich der besondere Vorteil, dass sowohl die Knochenschrauben als auch die Verriegelungskörper mit demselben Schraubendreher betätigt werden können. Der Schraubendreher kann somit als ein Multifunktionsinstrument mit einem Arbeitsende mit drei Funktionen fungieren: Halten der Knochenschraube über eine Konusklemmung, Einschrauben der Schraube über ein Sternprofil, und Verriegeln des Rastmechanismus mit einem kleineren Antriebsprofil, wie z.B. einem Sechskant.

Wenn die Scheibe in einer flachen Vertiefung auf der Oberseite der zervikalen Platte aufgenommen ist, steht die Scheibe um ein entsprechend geringeres Maß von der Oberseite der zervikalen Platte vor, was der flachen Bauweise des zervikalen Plattensystems weiter zugutekommt. Dieser Überstand über die Platte kann noch weiter verringert werden, wenn die Drehachse des Verriegelungskörpers nicht senkrecht zur Oberfläche der zervikalen Platte angeordnet, sondern um einige Winkelgrade, vorzugsweise im Bereich zwischen 3 und 7° zur Oberflächennormalen geneigt wird.

Dabei genügt es, wenn der Rastpin lediglich eine Höhe im Bereich zwischen 0,25 und 0,75 mm hat.

Die vorstehend beschriebene Verriegelung erlaubt es, die anteriore zervikale Platte mit Aufnahmedurchbrüchen für die Knochenschrauben in Form von Bohrungen und Langlöchern auszustatten, wodurch sich das zervikale Plattensystem für alle gängigen Versorgungsoptionen eignet.

Ein besonders materialsparendes Design des Verriegelungskörpers ergibt sich dann, wenn der Verriegelungskörper im MIM-Verfahren (Metal Injection Molding), vorzugsweise aus einer Ti6Al4V oder CoCr-Legierung hergestellt ist. Mit dieser Materialwahl kann selbst bei sehr geringer Dicke der Scheibe des Verriegelungskörpers eine ausreichende Festigkeit, sowie ein geeigneter Rastmechanismus realisiert werden.

Nachstehend werden anhand schematischer Zeichnungen mehrere Ausführungsbeispiele des neuen Systems mit anteriorer zervikaler Platte und einer Verriegelung für eingesetzte, mit einem Wirbelkörper verschraubte und die zervikale Platte gegen den Wirbelkörper drückende Knochenschrauben näher beschrieben. Es zeigen:

### Kurzbeschreibung der Figuren

Fig. 1 eine Unteransicht einer ersten Ausführungsform einer anterioren zervikalen Platte mit montierten Verriegelungskörpern für eingesetzte Knochenschrauben;
Fig. 2 die Seitenansicht der in Figur 1 gezeigten zervikalen Platte;
Fig. 3 die Draufsicht der zervikalen Platte mit montierten Verriegelungskörpern;
Fig. 4 die Schnittansicht gemäß IV-IV in Figur 3;
Fig. 5 die Draufsicht eines bei der Ausführungsform gemäß Fig. 1 bis 4 verwendeten Verriegelungskörpers;
Fig. 6 die Seitenansicht des Verriegelungskörpers gemäß Fig. 5;
Fig. 7 die Ansicht des Verriegelungskörpers gemäß Fig. 5 von unten;
Fig. 8 die Schnittansicht gemäß VIII-VIII in Fig. 5;
Fig. 9 die Schnittansicht gemäß IX-IX in Fig. 7;
Fig. 10 die Schnittansicht gemäß X-X in Fig. 7;
Fig. 11 einen Ausschnitt einer schematischen Draufsicht der zervikalen Platte gemäß Fig. 1 bis 4 bei abgenommenem Verriegelungskörper;
Fig. 12 die Ansicht von unten;
Fig. 13 die vergrößerte Schnittansicht XIII-XIII gemäß Fig. 11;
Fig. 14 einen Ausschnitt der Fig. 13;
Fig. 15 eine Draufsicht einer weiteren Ausführungsform des zervikalen Plattensystems mit modifiziertem Verriegelungskörper;
Fig. 16 die Seitenansicht des Systems gemäß Fig. 15;
Fig. 17 die Draufsicht des bei der Ausführungsform gemäß Fig. 15 verwendeten Verriegelungskörpers bis Fig. 23 den Fig. 5 bis 10 entsprechende Ansichten und Schnittansichten des beim System gemäß Fig. 15 verwendeten Verriegelungskörpers;
Fig. 18 die Seitenansicht des Verriegelungskörpers gemäß Fig. 17;
Fig. 19 die Ansicht des Verriegelungskörpers gemäß Fig. 17 von unten;
Fig. 20 die Schnittansicht gemäß XX-XX in Fig. 17;
Fig. 21 die Schnittansicht gemäß XXI-XXI in Fig. 19;
Fig. 22 die Schnittansicht gemäß XXII-XXII in Fig. 19;
Fig. 23 die Schnittansicht gemäß XXIII-XXIII in Fig. 19;
Fig. 24 bis 27 den Figuren 11 bis 14 entsprechende Ansichten eines Ausschnitts der zervikalen Platte gemäß Fig. 15 bei abgenommenem Verriegelungskörper;
Fig. 28 bis 30 den Figuren 1 bis 3 entsprechende Ansichten eines weiteren Ausführungsbeispiels des zervikalen Plattensystems;
Fig. 31 eine perspektivische Ansicht des zervikalen Plattensystems gemäß Fig. 28 in einem Zustand in dem eine Knochenschraube mittels eines Multifunktionswerkzeugs gesetzt wird;
Fig. 32 eine Schnittansicht einer Knochenschraube mit eingesetztem Multifunktionswerkzeug;
Fig. 33 eine der Fig. 31 entsprechende Ansicht der zervikalen Platte mit angesetztem Multifunktionswerkzeug zur Verriegelung der gesetzten Knochenschrauben;
Fig. 34 eine Schnittansicht des im Einsatz befindlichen Multifunktionswerkzeugs gemäß Fig. 33;
Fig. 35 eine Draufsicht eines weiteren Ausführungsbeispiels des zervikalen Plattensystems mit modifizierter Kombination von Verriegelungskörpern bei gesetzten Knochenschrauben;
Fig. 36 die Seitenansicht des Systems gemäß Fig. 35;
Fig. 37 die Draufsicht eines weiteren Ausführungsbeispiels eines Verriegelungskörpers;
Fig. 38 die Seitenansicht der Verriegelungskörpers gemäß Fig. 37;
Fig. 39 die Ansicht des Verriegelungskörpers gemäß Fig. 37 von unten;
Fig. 40 die perspektivische Ansicht eines weiteren Ausführungsbeispiels des zervikalen Plattensystems;
Fig. 41 einen vergrößerten Ausschnitt der Ansicht gemäß Fig. 40;
Fig. 42 die Draufsicht der zervikalen Platte gemäß Fig. 40 bei abgenommenen Verriegelungskörpern;
Fig. 43 die Schnittansicht gemäß XLIII-XLIII in Fig. 42;
Fig. 44 in vergrößertem Maßstab der Längsschnitt durch einen Endabschnitt einer zervikalen Platte bei abmontiertem Verriegelungskörper;
Fig. 45 eine Längsschnittansicht mit montiertem Verriegelungskörper;
Fig. 46 eine vergrößerte Seitenansicht einer zervikalen Platte;
Fig. 47 eine vergrößerte perspektivische Ansicht der anterioren zervikalen Platte gemäß Fig. 1 mit einer Gegenüberstellung zweier unterschiedlich montierter Verriegelungskörper; und
Fig. 48 den Längsschnitt der Einzelheit gemäß Fig. 47.

### Beschreibung der Ausführungsbeispiele

Nachstehend werden Ausführungsbeispiele der vorliegenden Offenbarung auf der Basis der zugehörigen Figuren beschrieben.

### Erstes Ausführungsbeispiel

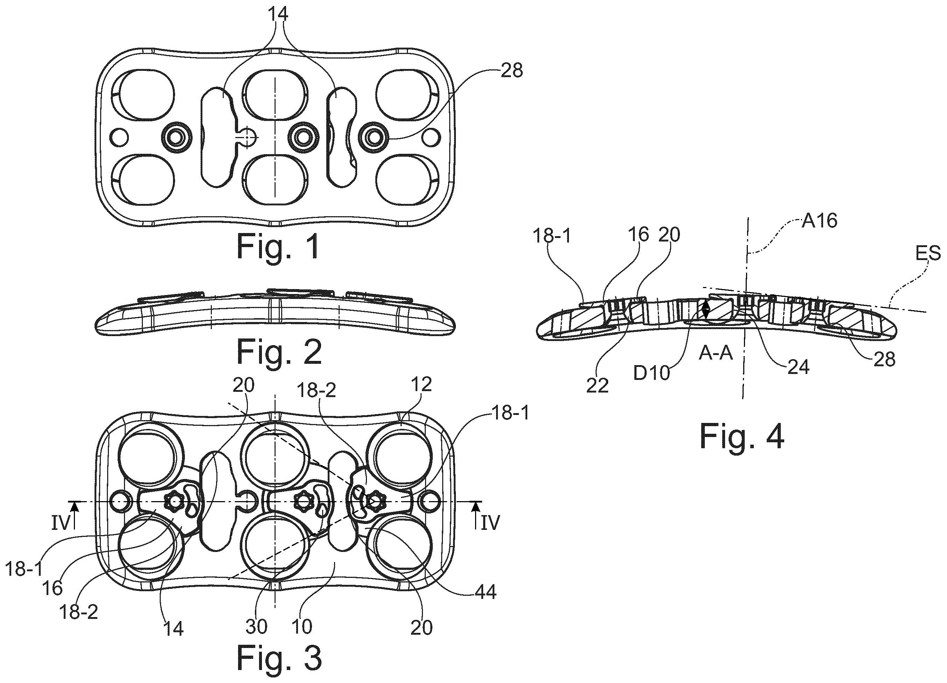

In den Figuren 1 bis 14 ist ein erstes Ausführungsbeispiel eines Systems mit anteriorer zervikaler Platte 10 mit 6 kreisrunden Aufnahmedurchbrüchen 12 für nicht näher dargestellte Knochenschrauben und einer Verriegelung für eingesetzte, mit einem Wirbelkörper verschraubte und die zervikale Platte gegen den Wirbelkörper drückende Knochenschrauben gezeigt. Neben den im Wesentlichen kreisrunden Aufnahmedurchbrüchen 12 hat die zervikale Platte 10 noch zwei Fenster 14. Anlageflächen für die Köpfe der Knochenschrauben sind nicht näher beschrieben, sie sind allerdings vorzugsweise so ausgebildet, dass sie - wie aus der Figur 3 ersichtlich - aus einer Kugelfläche in Kombination mit einer Kegelfläche bestehen, wodurch sowohl winkelmäßig eingeschränkte (constrained) Schrauben als auch winkelmäßig variable Schrauben verwendet werden können. Die gezeigte zervikale Platte 10 stellt damit eine sogenannte Zwei-Segment-Hybridplatte dar, bei der ein sogenanntes translatorisches Schraubengleiten verhindert ist. Alternativ können die Anlageflächen auch nur aus einer Kugelfläche oder nur einer Kegelfläche bestehen.

In an sich bekannter Weise ist die zervikale Platte 10 in Längsrichtung vorgebogen und in Querrichtung leicht gewölbt, um sich möglichst eng an die Form der Halswirbelsäule anzuschmiegen.

Die zervikale Platte 10 ist mit einem im Folgenden näher zu beschreibenden Verriegelungsmechanismus für die gesetzten und mit den Halswirbeln verschraubten Knochenschrauben ausgestattet. Durch diesen Verriegelungsmechanismus werden die Knochenschrauben an einer Migration der Schrauben aus der Platte heraus gehindert.

Der Verriegelungsmechanismus muss so ausgebildet sein, dass ein Verriegelungskörper 16 aus einer einen Aufnahmedurchbruch 12 frei zugänglich lassenden Position in eine Verriegelungsposition bringbar ist, in der der im Aufnahmedurchbruch 12 liegende Schraubenkopf vom Verriegelungskörper 16 zumindest teilweise abgedeckt ist und so die Migration der Schraube aus der zervikalen Platte 10 heraus verhindert wird.

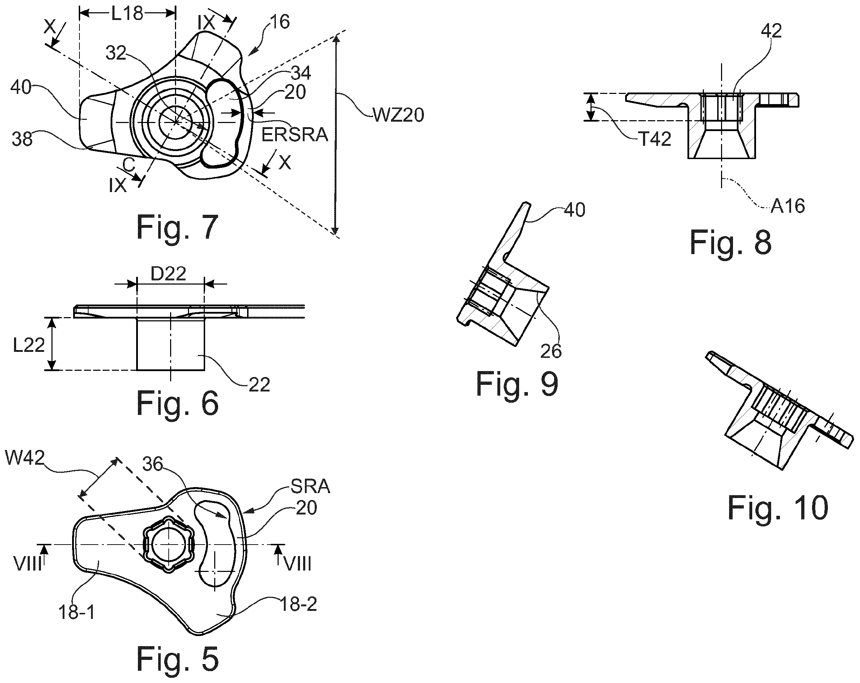

Im gezeigten Ausführungsbeispiel sind drei Verriegelungskörper 16 vorgesehen, die jeweils zwei benachbarten Aufnahmedurchbrüchen 12 zugeordnet und jeweils im Wesentlichen mittig zwischen zwei benachbarten Aufnahmedurchbrüchen 12 angeordnet sind. Jeder Verriegelungskörper 16 ist von einer drehbar um eine im Wesentlichen senkrecht auf der zervikalen Platte 10 stehende Drehachse A16 an der anterioren zervikalen Platte 10 festgelegten dünnen Scheibe 16 gebildet, die über den Umfang winkelmäßig verteilt zumindest einen Verriegelungsarm 18-1 und 18-2 und einen sich um einen Zentriwinkel WZ20 (siehe Fig. 7) erstreckenden Stützabschnitt 20 aufweist, der in der Scheibenebene liegt und durch Verdrehen der Scheibe 16 um einen vorbestimmten Drehwinkel WD, der in Figur 3 mit den beiden gestrichelten Linien angedeutet ist und im Wesentlichen dem Zentriwinkel WZ20 entspricht, aus der in Figur 3 dargestellten geöffneten Position in eine die Knochenschrauben verriegelnde Rastposition schnappt.

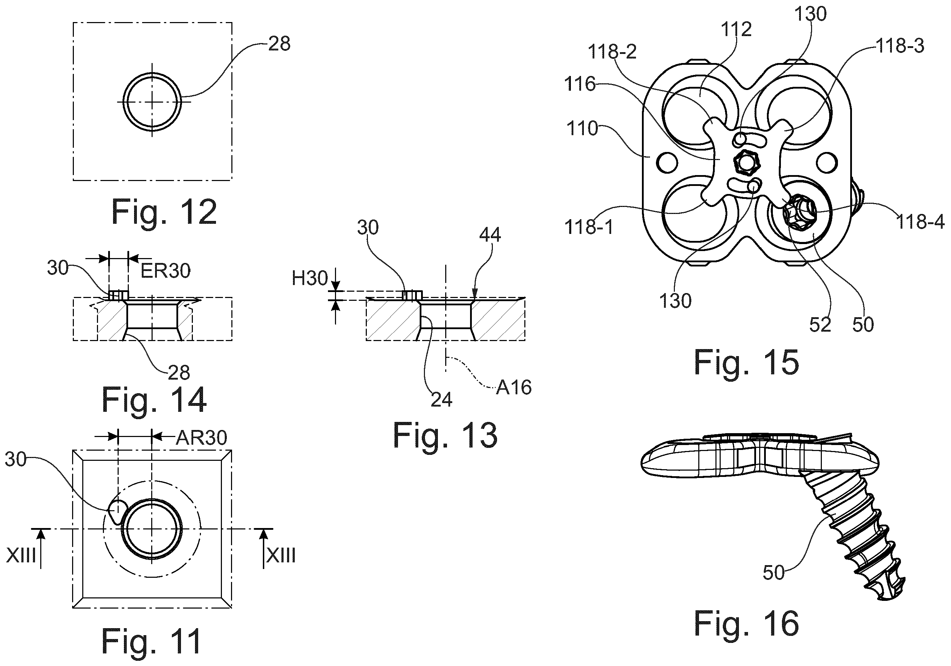

Die Verdrehbarkeit der Scheibe 16 wird durch einen am besten in den Figuren 6 und 8 bis 10 dargestellten, vorzugsweise einstückig mit der Scheibe 16 ausgebildeten Lagerzapfen 22 bereitgestellt, der mit Spielpassung in einer Bohrung 24 der zervikalen Platte 10 aufgenommen ist. Der Lagerzapfen 22 mit einem Durchmesser D22 und einer Länge L22, die im Wesentlichen der Dicke D10 (siehe Fig.4) der zervikalen Platte 10 entspricht, ist hohlzylinderförmig derart ausgebildet, dass sich seine Wandstärke - wie am besten den Fig. 8 bis 10 entnehmbar - zu seinem der Rückseite der zervikalen Platte zugewandten, d.h. der Scheibe 16 abgewandten Endabschnitt über einen sich aufweitenden Innenkonus 26 zunehmend verjüngt und dort mit der zervikalen Platte 10 über eine - in Figur 13 und 14 am besten gezeigte - Anfasung 28 der Bohrung 24 verstemmt ist. Der Verriegelungskörper in der Ausgestaltung als Scheibe 16 mit hohlem Lagerzapfen 22 ist auf diese Weise kanülliert, was dazu genutzt werden kann, den Lagerzapfen 22 als Führung für einen temporären Fixationspin zum Fixieren der zervikalen Platte 10 an der Wirbelsäule heranzuziehen.

Die Abmessungen der Scheibe 16 sind so gewählt, dass sie die Gesamtdicke des zervikalen Plattensystem nur minimal anheben. Beispielsweise liegt die Dicke D16 (siehe Fig. 6) der Scheibe 16 für den Fall, dass die Dicke D10 der zervikalen Platte 10 zwischen 1,5 und 2,5 mm beträgt, im Bereich zwischen 0,25 und 0,75 mm. Der Durchmesser D22 des Lagerzapfens 22 liegt in diesem Fall im Bereich zwischen 1,2 und 3,5 mm.

Die Gesamtdicke des zervikalen Plattensystems kann dadurch noch etwas verringert werden, dass die Scheibe 16 in einer flachen Vertiefung 44 (siehe Figur 3 und 13) auf der Oberseite der zervikalen Platte 10 aufgenommen ist.

Um den in das zervikale Plattensystem integrierten Rastmechanismus bereitzustellen, wirkt der Stützabschnitt 20 mit einem auf der zervikalen Platte 10 angebrachten Rastpin 30 zusammen, der beim Verdrehen der Scheibe 16 den Stützabschnitt 20 vor Erreichen der Rastposition verformt, bevor die Scheibe 16 in die Rastposition schnappt.

Der Rastpin 30 hat - wie am besten in Figur 11 gezeigt - einen tropfenförmigen Querschnitt mit einer - in Figur 14 eingetragenen radialen Erstreckung ER30, die im Bereich zwischen 0,5 und 1,5 mm liegt. Bei dem gezeigten Ausführungsbeispiel liegt das Maß ER30 bei 0,9 mm. Die Höhe H30 (siehe Figur 13) des Rastpins 30 entspricht im Wesentlichen der Dicke D16 der Scheibe 16.

Im gezeigten Ausführungsbeispiel ist der Stützabschnitt 20 - wie am besten aus den Figuren 5 und 7 gezeigt - von einem im Wesentlichen kreisbogenförmig verlaufenden Scheibenringausschnitt SRA gebildet ist, der beim Verdrehen der Scheibe 16 aus der in Figur 3 gezeigten Position vom Rastpin 30 in radialer Richtung verformt wird und bei Erreichen der - nicht gezeigten -Verriegelungsposition mit einer Rastmulde 32 (siehe Fig. 7) flächig auf den Rastpin 30 schnappt. Anders ausgedrückt ist der Rastpin 30 in einer bogenförmigen Kulisse 34 gefangen, in der er sich beim Verdrehen der Scheibe 16 zunächst mit Spiel bewegen kann, bevor er vor Erreichen der Rastmulde 32 auf eine vorspringende Nase 36 aufläuft, wodurch der Scheibenringausschnitt SRA nach außen gebogen wird.

Der Scheibenringausschnitt SRA hat eine - in Figur 7 bemaßte - kleine radiale Erstreckung ERSRA, die im Bereich eines mm-Bruchteils liegt.

Sobald beim Weiterdrehen der Scheibe 16 die Nase 36 überwunden ist, schnappt der Scheibenringausschnitt SRA auf den Rastpin 30 und schmiegt sich mit der Rastmulde 32 an diesen an. In dieser Position hat die Scheibe 16 die die Knochenschrauben verriegelnde Rastposition erreicht, in der die Verriegelungsarme 18-1 und 18-2 über die Aufnahmedurchbrüche 12 verschwenkt sind und diese teilweise verdecken. Die Rastmulde 32 ist hinsichtlich ihrer Form und Position an den Querschnitt des Rastpins angepasst, so dass das Öffnen ein bestimmtes Drehmoment erfordert und mittels Handkraft erfolgen kann.

Damit die vorstehend beschriebene Funktion des Verriegelungskörpers in Form der Scheibe 16 mit Lagerzapfen 22 mit möglichst geringem Fertigungsaufwand hergestellt werden kann, ist es vorteilhaft, ihn im MIM-Verfahren (Metal Injection Molding), vorzugsweise aus einer Ti6Al4V oder CoCr-Legierung herzustellen.

Einzelheiten der Gestaltung des Rastpins 30 sind den Figuren 11 bis 14 zu entnehmen. Es ist zu erkennen, dass der Rastpin 30 einen Querschnitt hat, der ein Gleiten innerhalb der Kulisse 34 begünstigt und geometrisch an die Rastmulde 32 derart angepasst ist, dass die Scheibe 16 leichtgängig in und aus der Schnappposition gebracht werden kann, wobei jedoch gleichzeitig dafür gesorgt sein soll, dass die Bedienungsperson das Einschnappen in die Verriegelungsposition spürt. Der Verriegelungszustand ist dadurch haptisch und auch optisch eindeutig zu erkennen.

Aus der vorstehenden Beschreibung wird deutlich, dass der gesamte Zentriwinkel WZ20 zur Bereitstellung der Verformung genutzt werden kann. Es wird somit ein Federelement bereitgestellt, das eine weiche Federkennlinie hat, wodurch Toleranzen an der zervikalen Platte 10 sowie am Verriegelungskörper, also der Scheibe 16 ausgeglichen werden können, ohne dass die zur Verriegelung und Entriegelung erforderlichen Momente negativ beeinflusst werden.

Diese Gestaltung des Rastmechansimus kommt der Bedienungsfreundlichkeit des Verriegelungskörpers zugute. Denn die Scheibe 16 kann leicht mit Handkraft, vorzugsweise mittels eines Bedienwerkzeugs, wie z.B. eines Schraubendrehers in die und aus der Verriegelungsposition gebracht werden, wobei der zur Verdrehung der Scheibe 16 dienende Antrieb so dimensioniert ist, dass das Verriegeln zu keiner Funktionsbeeinträchtigung führt, weil die Auslenkung bzw. Deformation des Rastmechanismus in radialer Richtung funktioniert.

Im gezeigten Ausführungsbeispiel wird zur Verdrehung der Scheibe 16 ein Werkzeug verwendet, das mit einer Mehrkantausnehmung im Lagerzapfen 22 zusammenwirkt. Den Figuren 8 bis 10 kann entnommen werden, dass die die Mehrkantausnehmung 42 aufgrund der recht kleinen erforderlichen Drehkräfte lediglich mit einer Tiefe T42 im mm-Bereich ausgebildet werden muss. Außerdem kann die Mehrkantausnehmung 42 mit einer kleinen Weite W42 (siehe Figur 5) ausgebildet werden, die ebenfalls im mm-Bereich liegt und damit kleiner ist als die Weite eines Klemmkonus am Schraubwerkzeug für die betreffenden Knochenschrauben.

Es hat sich gezeigt, dass dieser Zentriwinkel WZ20, der im Wesentlichen dem Verdrehwinkel der Scheibe 16 aus der einen Aufnahmedurchbruch 12 frei zugänglich lassenden Position in die Verriegelungsposition entspricht, in weiten Grenzen variieren und im Bereich zwischen 10° und 180° liegen kann.

Eine weitere Besonderheit des neuerungsgemäßen Verriegelungsmechanismus ist darin zu sehen, dass der Rastmechanismus einen sehr kleinen Platzbedarf hat. Denn der Rastpin 30 hat - wie am besten aus den Figuren 11 bis 14 hervorgeht - von der Drehachse A16 der Scheibe 16 einen Radialabstand AR30, der nur einen Bruchteil der Länge L18 (siehe Figur7) des Verriegelungsarms 18-1 bzw. 18-2 ausmacht. Mit dieser Anordnung können die Aufnahmedurchbrüche 12 und die Fenster 14 in der zervikalen Platte 10 möglichst groß ausgeführt werden, wobei sich der zusätzliche Vorteil ergibt, dass damit ein Aushebeln durch die Knochenschrauben zu vermeiden.

Bei dem vorstehend beschriebenen Ausführungsbeispiel sind 2 Verriegelungsarme 18-1, 18-2 vorgesehen. Die Scheibe 16 kann allerdings auch nur einen Verriegelungsarm oder auch bis zu vier Verriegelungsarme 18 ausbilden.

### Zweites Ausführungsbeispiel

In den Figuren 15 bis 27 ist ein zweites Ausführungsbeispiel eines zervikalen Plattensystems gezeigt. Zur Vereinfachung der Beschreibung sind Komponenten, die entsprechenden Bauteilen und Abschnitten des ersten Ausführungsbeispiels entsprechen, mit Bezugszeichen versehen, denen eine "1" vorangestellt ist.

Die zervikalen Platte 110 dieses Ausführungsbeispiels hat für die Knochenschrauben 50 nur vier Aufnahmedurchbrüche 112, die in der in Figur 15 gezeigten Verriegelungsstellung von einem einzigen Verriegelungskörper in der Ausgestaltung als Scheibe 116 mit vier Verriegelungsarmen 118-1 bis 118-4, die im Wesentlichen identisch ausgebildet sind, teilweise abgedeckt werden. Diese teilweise Abdeckung genügt, um die gesetzten Knochenschrauben 50 in ihrer Position zu halten und an einer Migration aus der zervikalen Platte 110 heraus zu hindern. Gleichzeitig lässt die Figur 15 erkennen, dass die teilweise Abdeckung der Aufnahmedurchbrüche 112 dazu führt, dass der Antrieb der Knochenschrauben 50 in der Ausgestaltung eines geeigneten Schrauben-Mitnahmeprofils, wie z.B. eines Sternprofils 52, noch zugänglich bleibt, wodurch ein Nachziehen der Schrauben auch in der Verriegelungsposition der Verriegelungsarme 118 möglich ist.

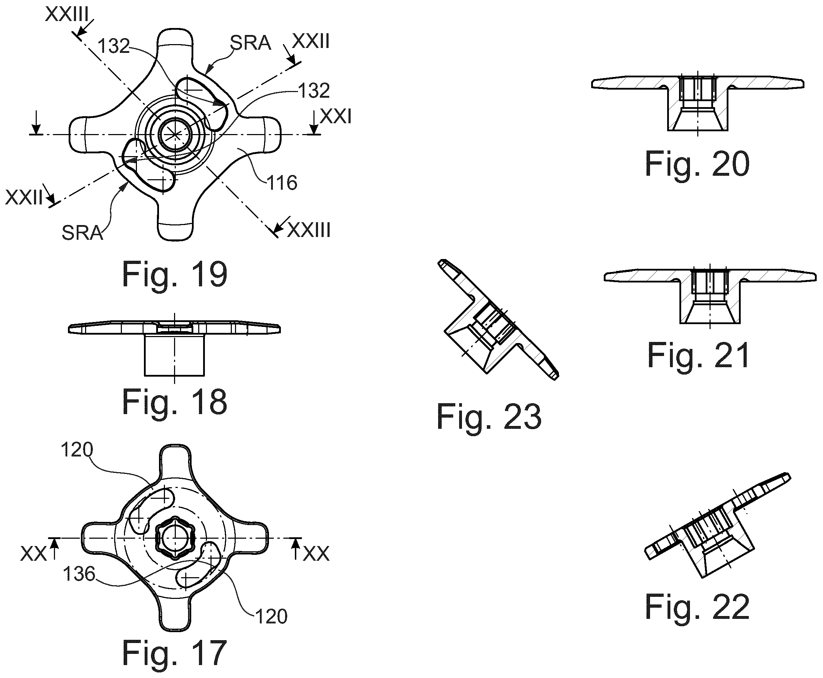

Die zur Verriegelung dienende Scheibe 116 ist im Detail in den Figuren 17 bis 23 dargestellt und ist, was ihre Materialwahl, Dimensionierungen hinsichtlich Dicke und Länge der Verriegelungsarme 118 sowie der Gestaltung des Lagerzapfens 122 anbelangt, mit der Scheibe 16 des ersten Ausführungsbeispiels vergleichbar, so dass auf eine erneute Beschreibung verzichtet werden kann.

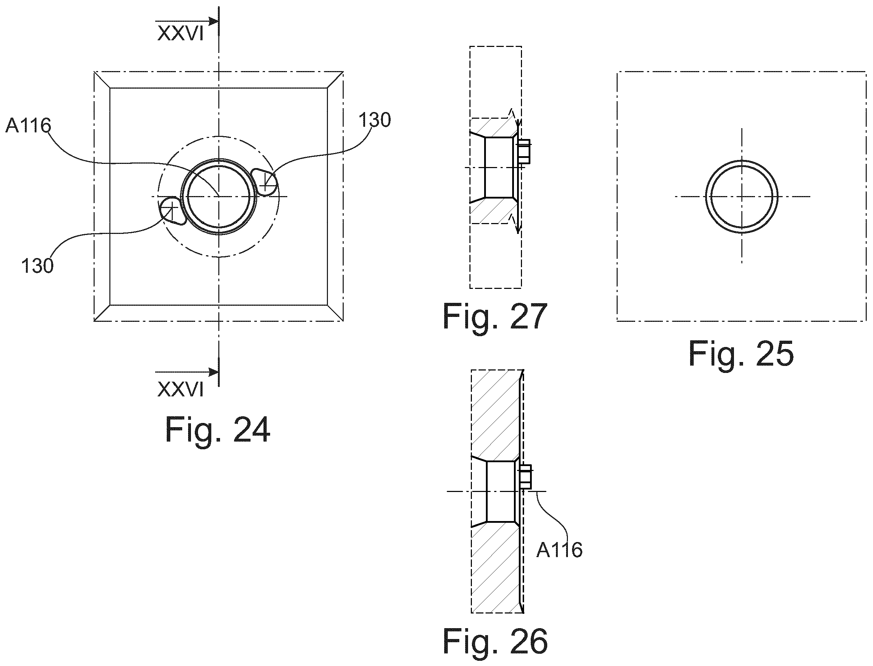

Abweichend vom ersten Ausführungsbeispiel trägt die zervikalen Platte 110 - wie am besten aus der Draufsicht gemäß Figur 24 gezeigt - zwei Rastpins 130, die - mit zwei punktsymmetrisch zueinander positionierten Stützabschnitten 120 (siehe Figur 17 und 19) zusammenwirken. Die Stützabschnitte 120 sind wie bei dem ersten Ausführungsbeispiel jeweils von einem im Wesentlichen kreisbogenförmig verlaufenden Scheibenringausschnitt gebildet ist, der beim Verdrehen der Scheibe 116 aus einer nicht gezeigten Position vom zugeordneten Rastpin 130 in radialer Richtung verformt wird und bei Erreichen der - in Figur 15 gezeigten -Verriegelungsposition mit einer Rastmulde 132 (siehe Fig. 19) flächig auf den Rastpin 130 schnappt. Der Rastpin 130 ist also ebenso wir beim ersten Ausführungsbeispiel in einer bogenförmigen Kulisse 134 gefangen, in der er sich beim Verdrehen der Scheibe 116 zunächst mit Spiel bewegen kann, bevor er vor Erreichen der Rastmulde 132 auf eine vorspringende Nase 136 aufläuft. Dabei wird der Scheibenringausschnitt SRA nach außen gebogen, bevor beim Weiterdrehen der Scheibe 116 die Nase 136 überwunden wird und er auf den Rastpin 130 schnappt, so dass er sich mit der Rastmulde 132 an letzteren anschmiegt. Die Rastmulde 132 ist hinsichtlich ihrer Form und Position wie bei dem ersten Ausführungsbeispiel so an den Querschnitt des Rastpins 130 angepasst, dass die Rastposition sicher gehalten wird und nur durch Überwindung eines vorbestimmten Haltemoments wieder aufgehoben werden kann.

Auch die Rastpins 130 sind - wie aus den Figuren 24 bis 27 gezeigt - hinsichtlich ihrer Lagezuordnung zur Drehachse A116 im Wesentlichen identisch zu dem ersten Ausführungsbeispiel gestaltet, so dass eine nähere Beschreibung entfallen kann.

### Drittes Ausführungsbeispiel

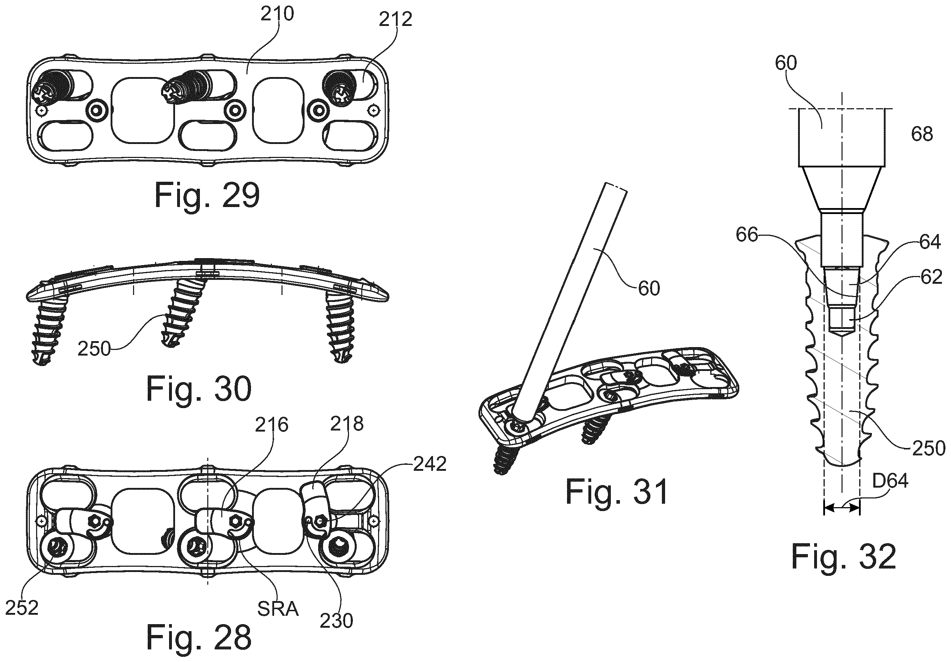

In den Figuren 28 bis 34 ist ein drittes Ausführungsbeispiel eines neuerungsgemäßen zervikalen Plattensystems gezeigt. Zur Vereinfachung der Beschreibung sind auch hier Komponenten, die entsprechenden Bauteilen und Abschnitten des ersten Ausführungsbeispiels entsprechen, mit Bezugszeichen versehen, denen eine "2" vorangestellt ist.

Abweichend von den zuvor beschriebenen Ausführungsbeispielen ist die zervikale Platte 210 als sogenannte dynamische Zwei-Segment-Platte ausgeführt. Sie hat neben zwei Fenstern 214 Aufnahmedurchbrüche 212, die als Langlöcher ausgebildet und so geformt sind, dass sie neben - nicht gezeigten - sogenannten constrained Schrauben mit kegeligem Schraubenkopf auch sogenannte variable Schrauben 250 mit sphärischem Schraubenkopf aufnehmen können.

Die Verriegelungskörper sind erneut als Scheibe 216 ausgestaltet, haben allerdings nur einen Verriegelungsarm 218, der in der Position dargestellt ist, in der die Aufnahmedurchbrüche 212 frei zugänglich sind, so dass die Knochenschrauben 250 gesetzt und mittels eines Werkzeugs 60 werden können.

Der Verriegelungsmechanismus entspricht demjenigen der oben beschriebenen Ausführungsbeispiele, so dass eine Beschreibung der Einzelheiten entfallen kann. Man erkennt aus der Darstellung gemäß Figur 28, dass die den Verriegelungskörper bildende Scheibe 216 durch den Eingriff eines Schraubendrehers in eine Mehrkantausnehmung 242 der Scheibe 216 in eine die Schrauben verriegelnde Rastposition verdreht werden kann, in der ein radial verformbarer Scheibenringausschnitt SRA auf den zugeordneten Rastpin 230 schnappt.

Anhand der Figuren 32 bis 34 wird beschrieben, wie das zur Betätigung der Knochenschrauben 250 dienende Werkzeug vorteilhafterweise ausgebildet ist, um die Handhabung des zervikalen Plattensystems möglichst bedienfreundlich zu gestalten.

Figur 32 zeigt, dass das Werkzeug 60 als Multifunktionswerkzeug ausgebildet ist und axial versetzt drei Funktionsabschnitte hat: an der Spitze einen Mehrkantabschnitt 62, z.B. Sechskantabschnitt, für den Eingriff in die Mehrkantausnehmung 242 des Verriegelungskörpers. Einen im Durchmesser D64 größeren Konusabschnitt 64 für den Passungseingriff mit einem Innenkonusabschnitt 66 der Schraube 250 und einen sich daran anschließenden, in der Weite den Konusabschnitt überragenden Sternprofilabschnitt 68 für den Eingriff in ein Schrauben-Mitnahmeprofil, vorzugsweise in ein Sternprofil 252 der Schraube 250.

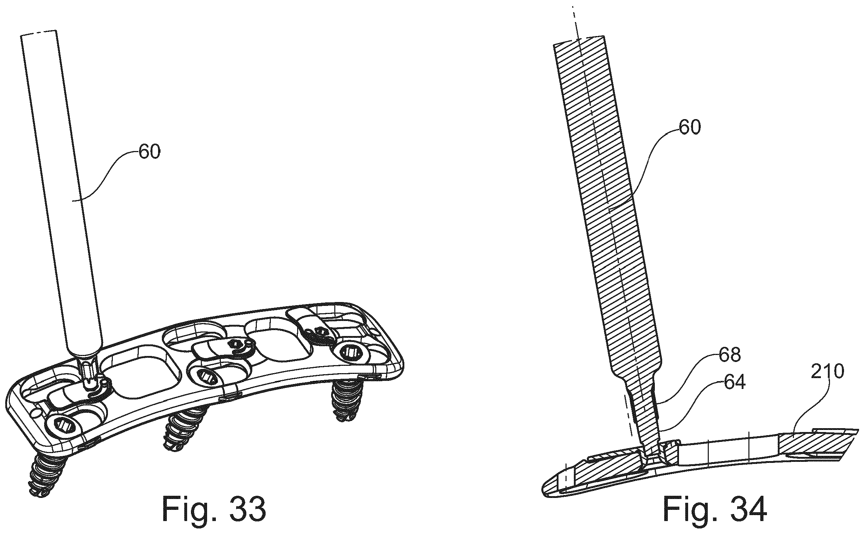

Figur 31 und 32 zeigt das Werkzeug 60 beim Verschrauben der zervikalen Platte 210. Figur 33 und 34 zeigen das Werkzeug 60 bei der Betätigung des Verriegelungsmechanismus. Hier ist nur der Mehrkantabschnitt 62 im Einsatz, während der Konusabschnitt 64 und der Sternprofilabschnitt 68 keine Funktion haben.

### Viertes Ausführungsbeispiel

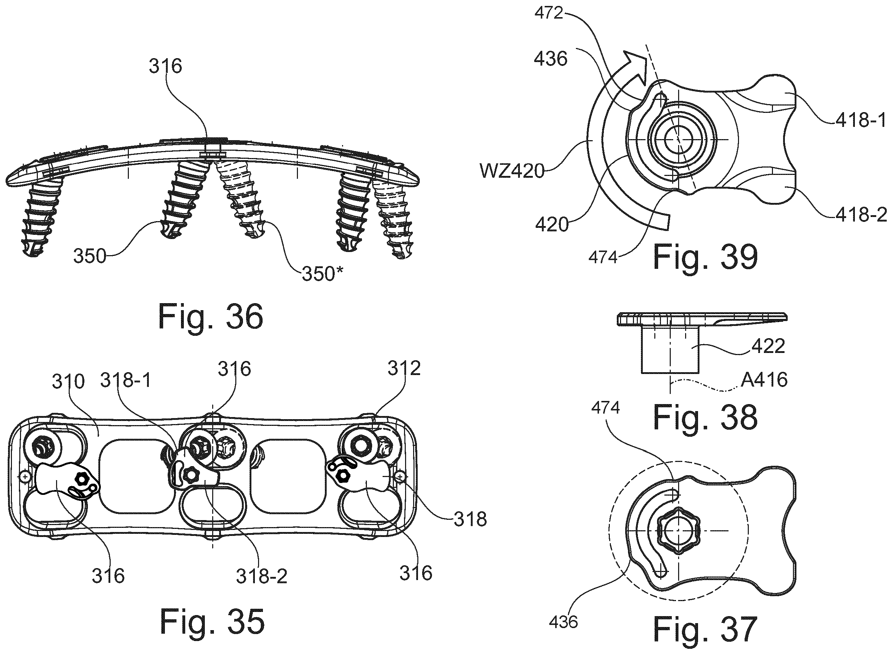

**In** den Figuren 35 und 36 ist ein viertes Ausführungsbeispiel eines neuerungsgemäßen zervikalen Plattensystems gezeigt. Zur Vereinfachung der Beschreibung sind auch hier Komponenten, die entsprechenden Bauteilen und Abschnitten des ersten Ausführungsbeispiels entsprechen, mit Bezugszeichen versehen, denen eine "3" vorangestellt ist.

Bei diesem Ausführungsbeispiel verwendet das zervikale Plattensystem eine zervikale Platte 310, die im Wesentlichen der zervikalen Platte 210 entspricht, also eine dynamische Zwei-Segment-Platte, in der die Schrauben 350 aufgenommen werden können. Durch die strichpunktiert dargestellten Schrauben 350* ist angedeutet, in welch großem Winkelbereich die Schrauben zur zervikalen Platte geneigt sein können. Abweichend vom dritten Ausführungsbeispiel ist die Platte 310 mit zwei verschiedenen Verriegelungskörpern kombiniert, die zwar jeweils als Scheibe 316, aber mit unterschiedlich gestalteten Verriegelungsarmen 318 bzw. 318-1 und 318-2 ausgebildet sind. Die nicht näher bezeichneten bogenförmigen Kulissen sind so gestaltet, dass ein für das Freigeben und Verriegeln der Schrauben ausreichend großer Verschwenkwinkel der Verriegelungskörper bereitgestellt ist.

### Fünftes Ausführungsbeispiel

Figur 37 bis 39 zeigt ein modifiziertes Ausführungsbeispiel eines beim neuerungsgemäßen zervikalen Plattensystems verwendbaren Verriegelungskörpers. Zur Vereinfachung der Beschreibung sind auch hier Komponenten des Verriegelungskörpers, die entsprechenden Bauteilen und Abschnitten des ersten Ausführungsbeispiels entsprechen, mit Bezugszeichen versehen, denen eine "4" vorangestellt ist.

Der Verriegelungskörper ist wieder als um eine Drehachse A416 beweglich in einer zervikalen Platte gelagerte Scheibe 416 mit Lagerzapfen 422 und zwei Verriegelungsarmen 418-1 und 418-2 ausgebildet. Der für den Funktionseingriff mit einem nicht gezeigten Rastpin vorgesehene Stützabschnitt 420 in der Ausgestaltung als Scheibenringausschnitt SRA erstreckt sich aber im Vergleich zu den oben beschriebenen Ausführungsbeispielen über einen größeren Zentriwinkel WZ420 (siehe Figur 39) der nahezu 180° beträgt, wodurch sich eine noch größere Länge des verformbaren Stützabschnitts 420 ergibt. In diesem Fall ist der nicht näher gezeigte Rastpin nicht in einer Kulisse gefangen. Stattdessen bewegt sich der nicht gezeigte Rastpin beim Verschwenken der Scheibe 416 in die Verriegelungsposition, vorzugsweise mit geringem Spiel, entlang der Außenseite des verformbaren Stützabschnitts 420 relativ zur Scheibe 416. Der verformbare Stützabschnitt bildet eine radial nach aussen gerichtete, beidseitig abgerundete Nase 436 (siehe Figur 39) aus, wodurch der Stützabschnitt 420 vor Erreichen der Verriegelungsposition radial nach innen verformt wird und beim Weiterdrehen in die Verriegelungsposition wieder zurückfedert, in der der Rastpin in einer Rastmulde 472 ruht.

Wenn die Scheibe 416 in die offene Position verdreht wird, bewegt sich die Nase 436 zunächst unter dem Rastpin unter kurzzeitiger Verformung des Stützabschnitts 420 hinweg. Sie Scheibe kann dann über den Zentriwinkel WZ420 kraftfrei verschwenkt werden, bis der Rastpin 30 einen Anschlag 474 bzw. eine Anschlagmulde erreicht. In dieser Position sind die beiden Verriegelungsarme 418-1 und 418-2 so weit verschwenkt, dass sie die Aufnahmedurchbrüche nicht mehr überlappen.

### Sechstes Ausführungsbeispiel

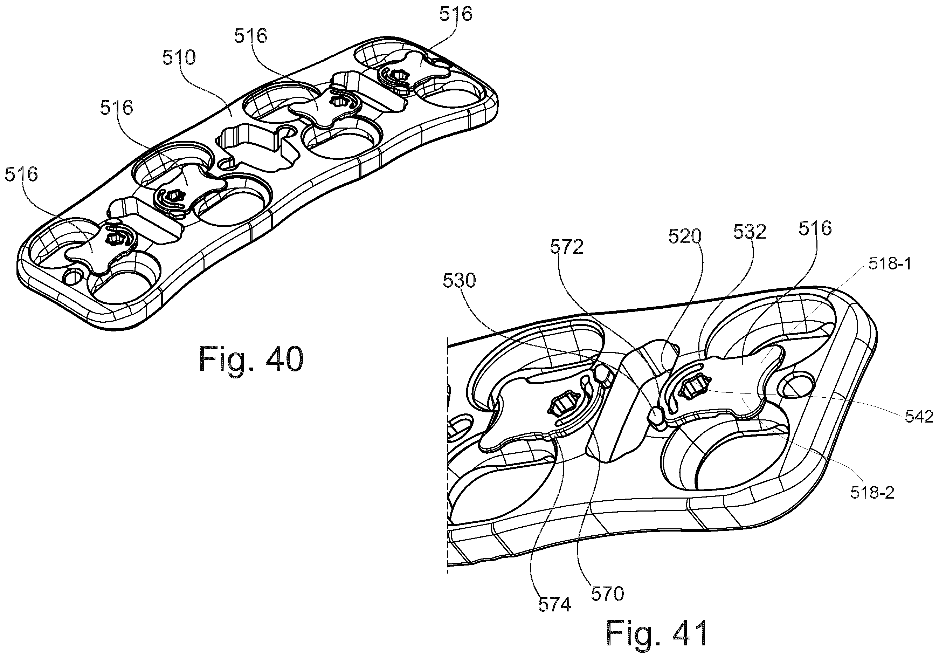

Figur 40 und 41 zeigt ein weiter modifiziertes Ausführungsbeispiel eines zervikalen Plattensystems mit variierter zervikaler Platte 510 und dem unter Bezug auf die Figuren 37 bis 39 beschriebenen Verriegelungskörper, also einem Verriegelungskörper in der Ausgestaltung als drehbar auf der zervikalen Platte 510 gelagerte Scheibe 516. Zur Vereinfachung der Beschreibung sind auch hier Komponenten der zervikalen Platte und des Verriegelungskörpers, die entsprechenden Bauteilen und Abschnitten der oben beschriebenen Ausführungsbeispiele entsprechen, mit Bezugszeichen versehen, denen eine "5" vorangestellt ist.

Die zervikale Platte 510 ist als dynamische 3-Segment-Platte ausgebildet, die somit mit vier Verriegelungskörpern in der Ausgestaltung als drehbar gelagerte Scheiben 516 bestückt ist. Die Form der Scheiben 516 stimmt mit derjenigen des Ausführungsbeispiels gemäß Figur 37 bis 39 überein. Der Stützabschnitt 520 wird vom Rastpin 530 in der unter Bezug auf die Figuren 37 bis 39 beschriebenen Art und Weise verformt.

In den Figuren 40 und 41 ist die Scheibe 516 in der verriegelten Stellung gezeigt, in der die beiden Verriegelungsarme 518-1 und 518-2 die (nicht gezeigten, gesetzten Schrauben) Schrauben zumindest teilweise abdecken. In dieser Verriegelungsposition ruht der Rastpin 530 in der Rastmulde 572, die hinter der Nase 536 liegt.

Aus dieser Schnappposition kann die Scheibe 516 mittels eines in die Mehrkantausnehmung 542 eingreifenden Werkzeugs in die geöffnete Position verdreht werden. Der Rastpin 530 bewegt sich dabei relativ zum Stützabschnitt 520 unter der Nase 536 hinweg, wobei sich dabei der Stützabschnitt 520 radial verformt. Anschließend, also nach Überwindung der Nase 536 kann die Scheibe 516 weiter vorzugsweise weitgehend kraftfrei, also mit geringfügigem Spiel zwischen Rastpin 530 und Stützfläche 570, verschwenkt werden, bis die Anschlagmulde 574 den Rastpin 530 erreicht.

Wenn die Scheibe 516 mittels eines in die Mehrkantausnehmung 542 eingreifenden Werkzeugs in die Verriegelungsposition verdreht wird, wird der Stützabschnitt 520 bei Erreichen der Nase 536 zunächst radial nach innen verformt. Bei erreichen der Verriegelungsposition schnappt der Stützabschnitt 520 mit der Rastmulde 572 auf den Rastpin 530, wodurch der Bedienungsperson haptisch und optisch das Erreichen der Verriegelungsposition signalisiert wird.

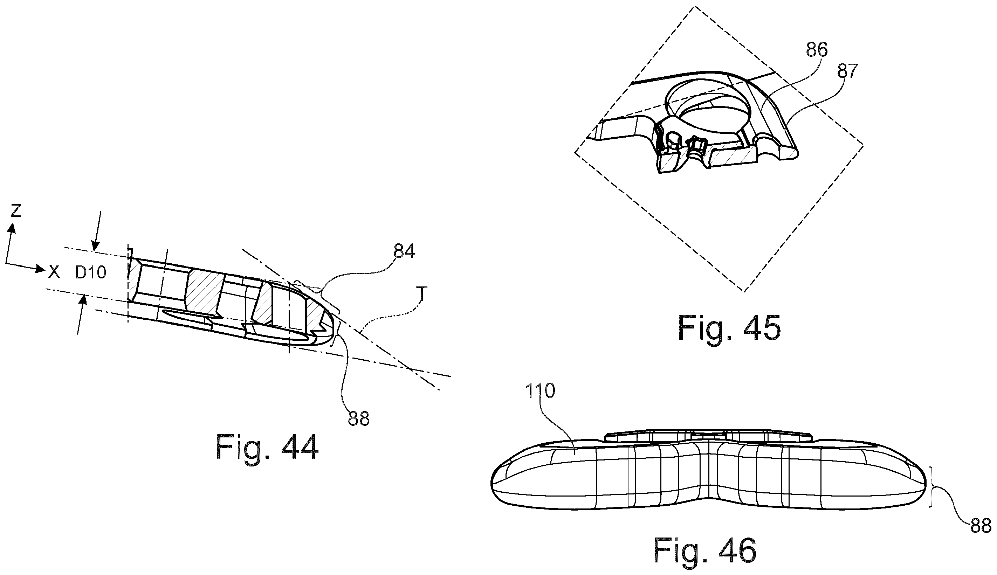

Diese neuerungsgemäße Dimensionierung des zervikalen Endabschnitts der zervikalen Platte kann - wie in Figur 46 dargestellt - vorteilhaft für alle Plattentypen Anwendung finden, also auch für eine Ein-Segment-Platte 110, wie sie in Figur 15 und 16 gezeigt ist.

### Weitere Aspekte

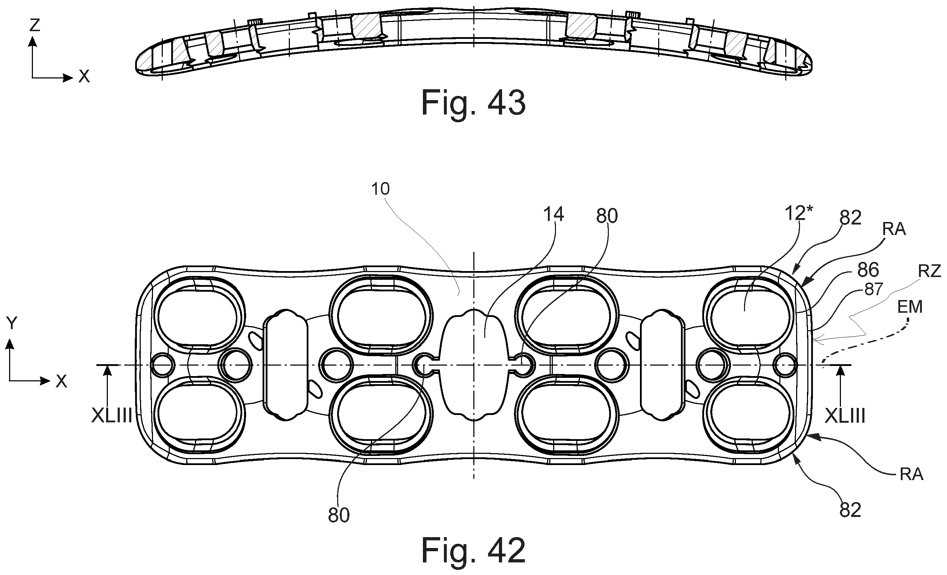

Anhand der Figuren 42 bis 46 werden weitere Besonderheiten des zervikalen Plattensystems beschrieben.

Eine erste Besonderheit besteht darin, dass die zervikale Platte 10 im Zentrumsbereich, also im Bereich des zentralen Fensters 14 zwei schlüssellochförmige, also hinterschnittene, zum Fenster 14 offene Ausnehmungen 80 aufweist, mit denen die zervikale Platte an ein chirurgisches Einsetzinstrument gekoppelt werden kann. Dadurch ergibt sich der Vorteil, dass da Einsetzinstrument nicht mehr über den Rand der zervikalen Platte vorsteht, wodurch Kollisionen des Instruments mit Knochen oder mit der Operationswunde vermieden werden können. Gleichzeitig können diese Ausnehmungen 80 für das Setzen von temporären Fixationspins herangezogen werden, mit denen eine einfache und exakte Ausrichtung der zervikalen Platte an der Halswirbelsäule unterstützt wird. Die Ausnehmungen 80 können zudem an der Unterseite der Platte hinterschnitten sein, um die Anbringung eines Einsetzinstrumentes zu ermöglichen.

Die weitere Besonderheit liegt in einer neuen Gestaltung des kranialen Endabschnitts der zervikalen Platte. Man erkennt aus den Darstellungen der Figuren 42 bis 46, dass sich die zervikale Platte im Bereich des kranialen Endabschnitts in Längsrichtung, also in Richtung der X-Achse gemäß Figur 42 verjüngt. Dabei nehmen in Längsrichtung sowohl die Breite der Platte (ΔY) als auch die Dicke der Platte (ΔZ) ab. In Querrichtung, also in Richtung der Y-Achse, ist eine Verrundung 82 mit den Radien RA und RZ bis zur Mittelebene EM der Platte vorgesehen, die sich mit einem in Z-Richtung gerichteten Verjüngungsabschnitt 84 (siehe Figur 44) entlang der X-Achse überlagert. Die zervikale Platte, die mit einer kleinen Dicke im Bereich zwischen 1,5 und 2,5 mm ausgeführt ist, verjüngt sich in Z-Richtung beginnend im Bereich des kranialen Aufnahmedurchbruchs 12* zunächst leicht bogenförmig und beginnend mit einer ersten Kante 86 (siehe Figur 42 und 45) linear (siehe Figur 42, 44 und 45), was in Figur 44 durch die Tangente T gezeigt ist, bis an einer zweiten geraden Kante 87 ein Rundungsabschnitt 88 mit einer Dicke D88 erreicht ist, wobei Dicke D88 nur noch 60% der Dicke D10 (siehe Figur 44, in der die mit strichpunktierten Linien gezeigten Bezugslinien auf der unteren bzw. oberen Oberfläche der Platte liegen) der zervikalen Platte 10 ausmacht. Unter der Dicke D10 der Platte 10 wird dabei also die Rohdicke verstanden, also die Dicke der Platte, bevor die Auflageflächen für die Drehriegel bzw. die scheibenförmigen Verriegelungskörper gefräst werden. Im Bereich des Zentrums der Platte 10 beträgt diese Dicke beispielsweise 2 mm. Mit dieser Gestaltung gelingt es, post-operative Schluckbeschwerden wirksam zu reduzieren.

### Weitere Varianten:

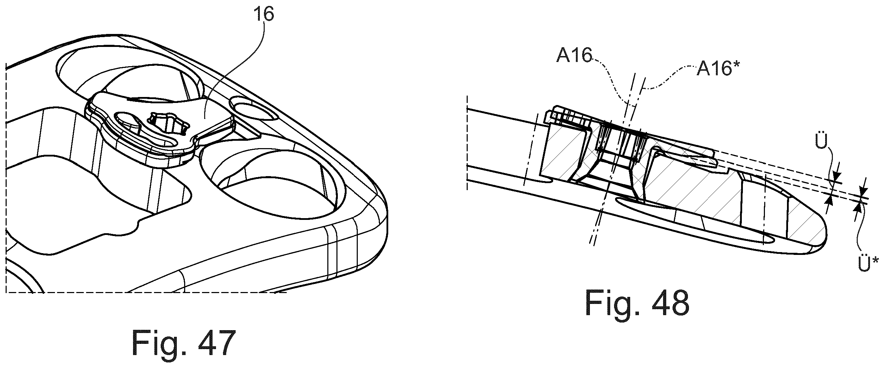

Vorstehend sind Verriegelungskörper in der Ausgestaltung als Scheiben beschrieben, die eine Drehachse haben, welche senkrecht auf der zervikalen Platte steht. In den Figuren 47 und 48 ist dargestellt, wie sich ein Überstand Ü der Scheibe 16 über die Oberfläche der zervikalen Platte 10 weiter verringern lässt, indem die Drehachse A16 um nur wenige Winkelgrade, beispielsweise um 5° verschwenkt wird. Mit der verschwenkten Lage der Drehachse A16* ergibt sich eine Überstand Ü*, der erheblich gegenüber dem Maß Ü verkleinert ist.

Der Rastpin des Rastmechanismus kann beispielsweise einen symmetrischen (z.B. kreisförmigen) oder asymmetrischen (aus Kreissegmenten und Geraden zusammengesetzten, z.B. tropfenförmigen) Querschnitt haben.

### Bezugszeichenliste

- 10: zervikale Platte
- D10: Dicke von 10
- 12, 12*: Aufnahmedurchbrüche
- 14: Fenster
- 16, 16*: Scheibe
- A16: Drehachse
- D16: Dicke von 16
- 18, 18-1, 18-2: Verriegelungsarme
- L18: Länge von 18
- 20: Stützabschnitt
- WZ20: Zentriwinkel von 20
- 22: Lagerzapfen
- D22: Durchmesser
- L22: Länge
- 24: Bohrung
- 26: Innenkonus
- 28: Anfasung
- 30: Rastpin
- AR30: radialer Abstand von 30
- H30: Höhe von 30
- 32: Rastmulde
- 34: Kulisse
- 36: Nase
- 38: Anschrägung
- 40: Anschrägung
- 42: Mehrkantausnehmung
- W42: Weite von 42
- T42: Tiefe von 42
- 44: Vertiefung
- 50: Knochenschrauben
- 52: Sternprofil
- 60: Werkzeug
- 62: Mehrkantabschnitt
- 64: Konusabschnitt
- D64: Durchmesser
- 66: Innenkonusabschnitt
- 68: Stenprofilabschnitt
- 80: Ausnehmungen
- 82: Verrundung
- 84: Verjüngungsabschnitt
- 86: erste Kante
- 87: zweite Kante
- 88: Rundungsabschnitt
- D88: Dicke des Rundungsabschnitts
- 470: Außenoberfläche
- 472: Rastmulde
- 474: Anschlagmulde

- ES: Scheibenebene
- EM: Mittelebene
- WD: Drehwinkel
- SRA: Scheibenringausschnitt
- ERSRA: radiale Erstreckung von SRA
- Ü, Ü*: Überstand
- T: Tangente

## Patentansprüche

1. System mit anteriorer zervikaler Platte (10) mit Aufnahmedurchbrüchen (12) für Knochenschrauben (50) und einer Verriegelung für eingesetzte, mit einem Wirbelkörper verschraubte und die zervikale Platte gegen den Wirbelkörper drückende Knochenschrauben (50), wobei die Verriegelung einen plattenartigen, an der zervikalen Platte beweglich gehaltenen Verriegelungskörper aufweist, der aus einer einen Aufnahmedurchbruch (12) möglichst frei zugänglich lassenden Position in eine Verriegelungsposition bringbar ist, in der der im Aufnahmedurchbruch (12) liegende Schraubenkopf vom Verriegelungskörper abgedeckt ist und so die Migration der Schraube aus der zervikalen Platte heraus verhindert wird, wobei der Verriegelungskörper von einer drehbar um eine senkrecht auf der zervikalen Platte (12) stehende Drehachse (A16) an der anterioren zervikalen Platte festgelegten dünnen Scheibe (16) gebildet ist, die zumindest einen Verriegelungsarm (18) und einen in der Scheibenebene liegenden Stützabschnitt (20) aufweist, der durch Verdrehen der Scheibe (16) um einen vorbestimmten Drehwinkel in eine durch Gegendrehung der Scheibe (16) wieder aufhebbare Rastposition schnappt, **dadurch gekennzeichnet, dass** der Verriegelungsarm (18) und der zumindest eine Stützabschnitt (20) über den Umfang winkelmäßig verteilt sind und der Stützabschnitt (20) mit einem auf der zervikalen Platte (10) angebrachten Rastpin (30) zusammenwirkt, der beim Verdrehen der Scheibe (16) den Stützabschnitt (20) vor Erreichen der Rastposition verformt, indem der Stützabschnitt (20) von einem im Wesentlichen kreisbogenförmig verlaufenden Scheibenringausschnitt (SRA) gebildet ist, der beim Verdrehen der Scheibe (16) vom Rastpin (30) in radialer Richtung verformbar ist und eine Rastmulde (32) für den Rastpin (30) ausbildet.

2. System nach Anspruche 1, **dadurch gekennzeichnet, dass** der Verriegelungskörper im Wesentlichen mittig zwischen zumindest zwei Aufnahmedurchbrüchen (12) für die Knochenschrauben (50) angeordnet ist.

3. System nach Anspruch 1 oder 2, **dadurch gekennzeichnet, dass** die Scheibe (16) bis zu vier Verriegelungsarme (18) hat, mit denen Köpfe der gesetzten Knochenschrauben (50) teilweise überdeckbar sind.

4. System nach Anspruch 3, **dadurch gekennzeichnet, dass** die Verriegelungsarme (18) endseitig in radialer und/oder in Umfangsrichtung angefast sind.

5. System nach einem der Ansprüche 1 bis 4, **dadurch gekennzeichnet, dass** der Rastpin (30) von der Drehachse (A16) der Scheibe (16) einen Radialabstand (AR30) hat, der nur einen Bruchteil der Länge (L18) des Verriegelungsarms (18) ausmacht.

6. System nach einem der Ansprüche 1 bis 5, **dadurch gekennzeichnet, dass** der Drehwinkel der Scheibe (16) aus der einen Aufnahmedurchbruch (12) möglichst frei zugänglich lassenden Position in die Verriegelungsposition im Bereich zwischen 10° und 180° liegt.

7. System nach einem der Ansprüche 1 bis 6, **dadurch gekennzeichnet, dass** die Scheibe (16) eine Dicke (D16) im Bereich zwischen 0,25 und 0,75 mm hat.

8. System nach einem der Ansprüche 1 bis 7, **dadurch gekennzeichnet, dass** die Scheibe (16) mit Handkraft, vorzugsweise mittels eines Bedienwerkzeugs (60), wie z.B. eines Schraubendrehers in die und aus der Verriegelungsposition bringbar ist.

9. System nach einem der Ansprüche 1 bis 8, **dadurch gekennzeichnet, dass** die Scheibe (16) über einen in einer Bohrung (24) der zervikalen Platte (10) mit Spielpassung aufgenommenen, hohlzylinderförmigen Lagerzapfen (22), dessen Wandstärke sich zu seinem der Rückseite der zervikalen Platte (10) zugewandten Endabschnitt zunehmend verjüngt und dort mit der zervikalen Platte (10) vorzugsweise über eine Anfasung (28) der Bohrung (24) verstemmt ist, ein Drehlager für die Scheibe (16) ausbildet.

10. System nach Anspruch 9, **dadurch gekennzeichnet, dass** ein der Scheibe (16) zugewandter Abschnitt des Lagerzapfens (22) eine Mehrkantausnehmung (42) für den Eingriff eines Bedienwerkzeugs (60) hat.

11. System nach Anspruch 130, **dadurch gekennzeichnet, dass** die Mehrkantausnehmung (42) eine Weite (W42) hat, die kleiner ist als die Weite (Durchmesser D64) eines Klemmkonusabschnitts (64) an einem Schraubwerkzeug (60) für die Knochenschraube (50).

12. System nach einem der Ansprüche 1 bis 11, **dadurch gekennzeichnet, dass** die Scheibe (16) zumindest teilweise in einer flachen Vertiefung (44) auf der Oberseite der zervikalen Platte (10) aufgenommen ist.

13. System nach einem der Ansprüche 1 bis 12 **dadurch gekennzeichnet, dass** der Rastpin (30) eine Höhe im Bereich zwischen 0,25 und 0,75 mm hat.

14. System nach einem der Ansprüche 1 bis 13, **dadurch gekennzeichnet, dass** die anteriore zervikale Platte (10) als Aufnahmedurchbrüche (12) für die Knochenschrauben (50) Bohrungen und/oder Langlöcher aufweist.

15. System nach einem der Ansprüche 1 bis 14, **dadurch gekennzeichnet, dass** der Verriegelungskörper im MIM-Verfahren (Metal Injection Molding), vorzugsweise aus einer Ti6Al4V oder CoCr-Legierung hergestellt ist

## Claims

1. A system with an anterior cervical plate (10) with receiving apertures (12) for bone screws (50) and a lock for inserted bone screws (50) screwed to a vertebral body and pressing the cervical plate against the vertebral body, wherein the lock has a plate-like locking body held movably on the cervical plate, which can be brought from a position leaving a receiving aperture (12) as freely accessible as possible into a locking position in which the screw head located in the receiving aperture (12) is covered by the locking body and thus the migration of the screw out of the cervical plate is prevented, wherein the locking body is formed by a thin disc (16) fixed to the anterior cervical plate rotatably about an axis of rotation (A16) standing perpendicularly on the cervical plate (12), wherein the thin disc (16) has at least one locking arm (18) and a support section (20) located in the disc plane, which snaps in by rotating the disc (16) by a predetermined angle of rotation into a latching position which can be released again by counter-rotation of the disc (16), **characterized in that** the locking arm (18) and the at least one support section (20) are angularly distributed over the circumference and the support section (20) cooperates with a latching pin (30) attached to the cervical plate (10), which, during rotation of the disc (16), deforms the support section (20) before reaching the latching position, **in that** the support section (20) is formed by a disc ring cutout (SRA) running substantially in the form of a circular arc, which, during rotation of the disc (16), can be deformed in the radial direction by the latching pin (30) and forms a latching trough (32) for the latching pin (30).

2. The system according to claim 1, **characterized in that** the locking body is arranged substantially centrally between at least two receiving apertures (12) for the bone screws (50).

3. The system according to claim 1 or 2, **characterized in that** the disc (16) has up to four locking arms (18), with which heads of the set bone screws (50) can be partially covered.

4. The system according to claim 3, **characterized in that** the locking arms (18) are chamfered at the end in the radial and/or in the circumferential direction.

5. The system according to one of claims 1 to 4, **characterized in that** the latching pin (30) has a radial distance (AR30) from the axis of rotation (A16) of the disc (16), wherein the radial distance (AR30) makes up only a fraction of the length (L18) of the locking arm (18).

6. The system according to one of claims 1 to 5, **characterized in that** the angle of rotation of the disc (16) from the position leaving a receiving aperture (12) as freely accessible as possible into the locking position lies in the range between 10° and 180°.

7. The system according to one of claims 1 to 6, **characterized in that** the disc (16) has a thickness (D16) in the range between 0.25 and 0.75 mm.

8. The system according to one of claims 1 to 7, **characterized in that** the disc (16) can be brought into and out of the locking position by manual force, preferably via an operating tool (60), such as for example a screwdriver.

9. The system according to one of claims 1 to 8, **characterized in that** the disc (16) forms a rotary bearing for the disc (16) via a hollow cylindrical bearing pin (22) received in a bore (24) of the cervical plate (10) with a clearance fit, wherein the wall thickness of the bearing pin (22) increasingly tapers towards its end section facing the rear side of the cervical plate (10) and is caulked there to the cervical plate (10) preferably via a chamfer (28) of the bore (24).

10. The system according to claim 9, **characterized in that** a section of the bearing pin (22) facing the disc (16) has a polygonal recess (42) for the engagement of an operating tool (60).

11. The system according to claim 130, **characterized in that** the polygonal recess (42) has a width (W42) which is smaller than the width (diameter D64) of a clamping cone section (64) on a screwing tool (60) for the bone screw (50).

12. The system according to one of claims 1 to 11, **characterized in that** the disc (16) is received at least partially in a shallow depression (44) on the upper side of the cervical plate (10).

13. The system according to one of claims 1 to 12, **characterized in that** the latching pin (30) has a height in the range between 0.25 and 0.75 mm.

14. The system according to one of claims 1 to 13, **characterized in that** the anterior cervical plate (10) has bores and/or elongated holes as receiving apertures (12) for the bone screws (50).

15. The system according to one of claims 1 to 14, **characterized in that** the locking body is produced by the **MIM** method (metal injection molding), preferably from a Ti6Al4V or CoCr alloy.

## Revendications

1. Système avec une plaque cervicale antérieure (10) avec des ouvertures de réception (12) pour des vis à os (50) et un verrouillage pour des vis à os (50) insérées, vissées à un corps vertébral et pressant la plaque cervicale contre le corps vertébral, dans lequel le verrouillage présente un corps de verrouillage de type plaque maintenu de manière mobile au niveau de la plaque cervicale qui peut être amené d'une position qui laisse une ouverture de réception (12) aussi librement accessible que possible dans une position de verrouillage dans laquelle la tête de vis se trouvant dans l'ouverture de réception (12) est recouverte par le corps de verrouillage et empêchant ainsi la migration de la vis hors de la plaque cervicale, dans lequel le corps de verrouillage est formé par un disque (16) mince fixé de manière rotative autour d'un axe de rotation (A16) se trouvant perpendiculairement sur la plaque cervicale (12) au niveau de la plaque cervicale antérieure, disque qui présente au moins un bras de verrouillage (18) et une section d'appui (20) se trouvant dans le plan de disque qui s'enclenche par rotation du disque (16) selon un angle de rotation prédéterminé dans une position d'encliquetage de nouveau supprimable par contre-rotation du disque (16), **caractérisé en ce que** le bras de verrouillage (18) et l'au moins une section d'appui (20) sont répartis angulairement sur la périphérie et la section d'appui (20) coagit avec une broche d'encliquetage (30) montée sur la plaque cervicale (10) qui déforme la section d'appui (20) lors de la rotation du disque (16) avant d'atteindre la position d'encliquetage en formant la section d'appui (20) par une section d'anneau de disque (SRA) s'étendant sensiblement en arc de cercle qui est déformable lors de la rotation du disque (16) par la broche d'encliquetage (30) dans le sens radial et forme une cavité d'encliquetage (32) pour la broche d'encliquetage (30).

2. Système selon la revendication 1, **caractérisé en ce que** le corps de verrouillage est agencé sensiblement au milieu entre au moins deux ouvertures de réception (12) pour les vis à os (50).

3. Système selon la revendication 1 ou 2, **caractérisé en ce que** le disque (16) présente jusqu'à quatre bras de verrouillage (18) avec lesquels des têtes des vis à os (50) placées peuvent être partiellement recouvertes.

4. Système selon la revendication 3, **caractérisé en ce que** les bras de verrouillage (18) sont chanfreinés côté extrémité dans le sens radial et/ou dans le sens périphérique.

5. Système selon l'une quelconque des revendications 1 à 4, **caractérisé en ce que** la broche d'encliquetage (30) présente une distance radiale (AR30) par rapport à l'axe de rotation (A16) du disque (16) qui constitue seulement une fraction de la longueur (L18) du bras de verrouillage (18).

6. Système selon l'une quelconque des revendications 1 à 5, **caractérisé en ce que** l'angle de rotation du disque (16) de la position qui laisse une ouverture de réception (12) aussi librement accessible que possible à la position de verrouillage se situe dans la plage entre 10° et 180°.

7. Système selon l'une quelconque des revendications 1 à 6, **caractérisé en ce que** le disque (16) présente une épaisseur (D16) dans la plage entre 0,25 et 0,75 mm.

8. Système selon l'une quelconque des revendications 1 à 7, **caractérisé en ce que** le disque (16) peut être amené avec la force manuelle, de préférence au moyen d'un outil de commande (60) tel qu'un tournevis, dans et hors de la position de verrouillage.

9. Système selon l'une quelconque des revendications 1 à 8, **caractérisé en ce que** le disque (16) forme un palier rotatif pour le disque (16) par le biais d'un tourillon (22) en forme de cylindre creux, reçu dans un perçage (24) de la plaque cervicale (10) avec un ajustement mobile, tourillon dont l'épaisseur de paroi se rétrécit de plus en plus vers sa section d'extrémité tournée vers le côté arrière de la plaque cervicale (10) et y est de préférence matée avec la plaque cervicale (10) par un chanfrein (28) du perçage (24).

10. Système selon la revendication 9, **caractérisé en ce qu'**une section du tourillon (22) tournée vers le disque (16) présente un évidement polygonal (42) pour l'engagement d'un outil de commande (60).

11. Système selon la revendication 13, **caractérisé en ce que** l'évidement polygonal (42) présente une largeur (W42) qui est inférieure à la largeur (diamètre D64) d'une section de cône de serrage (64) au niveau d'un outil de vis (60) pour la vis à os (50).

12. Système selon l'une quelconque des revendications 1 à 11, **caractérisé en ce que** le disque (16) est reçu au moins partiellement dans une cavité (44) plate sur le côté supérieur de la plaque cervicale (10).

13. Système selon l'une quelconque des revendications 1 à 12, **caractérisé en ce que** la broche d'encliquetage (30) présente une hauteur dans la plage entre 0,25 et 0,75 mm.

14. Système selon l'une quelconque des revendications 1 à 13, **caractérisé en ce que** la plaque cervicale antérieure (10) présente des perçages et/ou trous oblongs comme ouvertures de réception (12) pour les vis à os (50).

15. Système selon l'une quelconque des revendications 1 à 14, **caractérisé en ce que** le corps de verrouillage est fabriqué avec le procédé MIM (moulage par injection de métal), de préférence en un alliage Ti6Al4V ou CoCr.
